# EUROPÄISCHE PATENTSCHRIFT

(11) **EP 0 396 882 B1**
(45) Veröffentlichungstag und Bekanntmachung des Hinweises auf die Patenterteilung: **09.03.1994**
(21) Anmeldenummer: 90105201.9
(22) Anmeldetag: 20.03.1990
(51) Int. Cl.: A61B 1/00, A61M 25/02

(54) **Fixiervorrichtung zum Festlegen von Faszienhaltefäden mit Dichtkegel für die offene Laparoskopie**
Fixing device for holding fascia-holding-threads, with a conical seal for laparoscopy
Dispositif de fixation d'assemblage de fils de maintien de bande avec un joint conique pour laparoscopie

(30) Priorität: 12.05.1989 DE 3915597
(43) Veröffentlichungstag der Anmeldung: 14.11.1990
(73) Patentinhaber: Richard Wolf GmbH, 75438 Knittlingen (DE)
(72) Erfinder: Boebel, Manfred, D-7136 Oetisheim (DE); Becker, Henning, Dr. med., D-6680 Neunkirchen (DE)
(74) Vertreter: Wilcken, Thomas, Dipl.-Ing.

(56) Entgegenhaltungen:
- DE-A- 3 011 069
- US-A- 3 817 251
- US-A- 3 822 697
- US-A- 4 533 349
- US-A- 4 650 473

## Beschreibung

Die Erfindung geht von einer Vorrichtung zum Festlegen von Faszienhaltefäden mit einem auf einer Trokarhülse verschiebbaren Dichtkegel für die offene Laparoskopie der DE-A-30 11 069 aus, durch die ein Laparoskop in die Körperhöhle eingeführt werden kann.

Die Trokarhülse weist mindestens zwei sich vorteilhaft gegenüberliegende Schlitzklemmen für die Befestigung der Faszienhaltefäden sowie einen frei längsverschiebbaren Dichtungskegel auf. Diese bekannte Trokarhülse mit Dichtkegel hat den Nachteil, daß nach dem Einsetzen in eine Bauchdeckenöffnung die Trokarhülse und der Dichtkegel eine bestimmte Position zueinander einnehmen , die nicht verändert werden darf, da die Faszienhaltefäden mit der Trokarhülse fest verbunden diese gemeinsam mit dem Dichtkegel in der Bauchdeckenöffnung festlegen. Eine Änderung der relativen Lage zwischen Dichtkegel und Trokarhülse ist nicht möglich, weil sonst die Festlegung in der Bauchdeckenöffnung und damit der gasdichte Abschluß der Bauchhöhle aufgegeben wird.

Die Aufgabe der Erfindung besteht darin, die Trokarhülse ohne einen unbeabsichtigten Gasverlust in der Bauchhöhle frei in axiale Positionen verschieben zu können, wie es für den Eingriff erforderlich ist.

Diese Aufgabe wird durch die Merkmale des Anspruches 1 gelöst.

In einer weiteren Ausführung der Erfindung wird die Fixiervorrichtung so ausgebildet, daß diese sowohl für die normale Laparoskopie als auch für Trokarhülsen unterschiedlichen Durchmessers verwendet werden kann.

Die Erfindung mit weiteren Vorteilen wird nachstehend anhand der Zeichnung erläutert. Es zeigen:

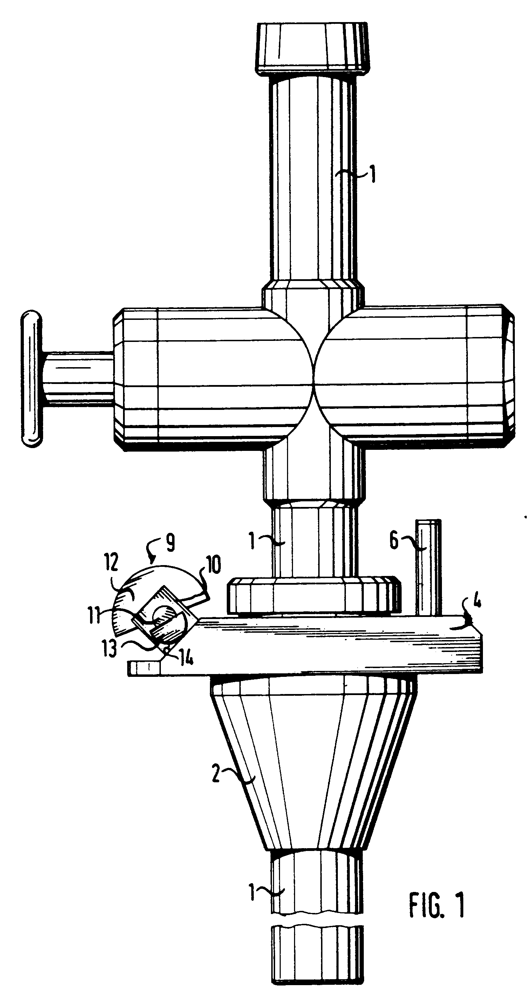

- Figur 1: die Seitenansicht einer Trokarhülse mit der aufgeschobenen, erfindungsgemäßen Fixiervorrichtung;
- Figur 2: einen Achsschnitt durch die Fixiervorrichtung nach der Erfindung;
- Figur 3: einen Achsschnitt durch die Fixiervorrichtung mit eingesetzter Reduzierhülse.

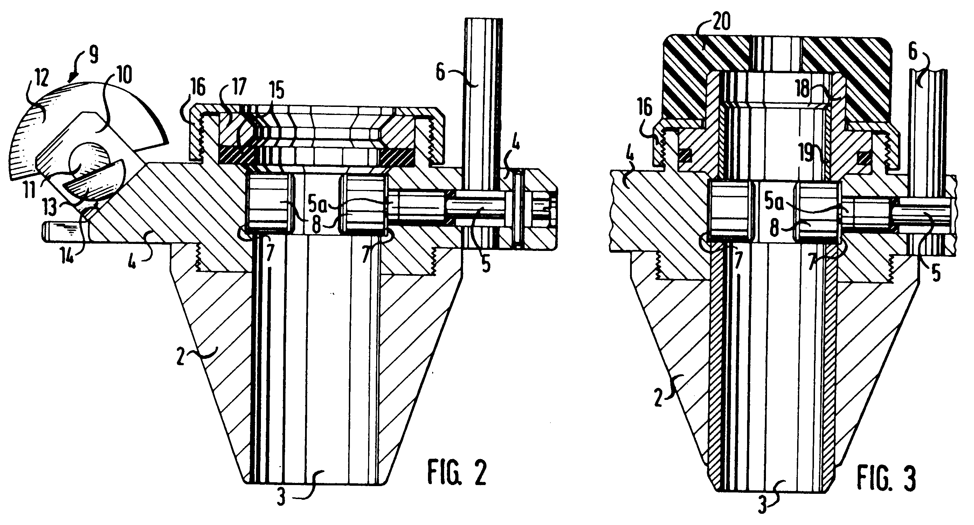

Auf die mit einem Ventil versehene Trokarhülse 1 ist die Fixiervorrichtung, bestehend aus dem Dichtkegel 2 und der Halteplatte 4, die einen zentralen Kanal 3 aufweisen, aufgeschoben. Der Dichtkegel 2 ist lösbar mit der oberen Halteplatte 4 verbunden, um diese auch für die normale Laparoskopie vervenden zu können. Zum Festlegen der Fixiervorrichtung auf der Trokarhülse 1 ist die Halteplatte 4 nach Figur 2 mit einer Radialbohrung für den Durchgriff einer abgedichtet axial verstellbaren Druck- und Zugstange 5 versehen. In das Gewinde der Stange 5 greift ein Gewinde in einer Querbohrung eines begrenzt verschwenkbaren Hebels 6 ein, der gegenüber der Stange 5 axial nicht verstellbar ist. Beim Verschwenken des Hebels 6 wird die Stange 5 in Richtung zum zentralen Kanal 3 über ein Ausgleichselement 5a gegen ein lose in einer flachen ringförmigen Ausnehmung 7 der Halteplatte 4 eingelegtes, unterbrochenes Spannelement 8 verschoben, durch das das freie Lumen um ein bestimmtes Maß verringert wird, so daß dadurch die Fixiervorrichtung 2,4 auf der durchgeführten Trokarhülse 1 leicht und bequem feststellbar und lösbar ist.

An mehreren Stellen der Halteplatte 4 sind Klemmvorrichtungen 9 für das Festlegen von Faszienhaltefäden vorgesehen, durch die die Fixiervorrichtung 2,4 in fester Lage zur Bauchdecke gehalten wird.

Die Klemmvorrichtungen 9 für die Faszienhaltefäden bestehen aus einem Lagerbock 10, in welchem eine verdrehbare Welle 11 gelagert ist, die nach Figur 2 auf einer Seite des Lagerbockes mit einer Verdrehhandhabe 12 und auf der anderen Seite exzentrisch ausgebildet ist oder mit einem Exzenter 13 versehen ist, dessen Umfangsfläche aufgerauht oder profiliert ist. Zwischen der Umfangsfläche des Exzenters 13 und der dieser zugekehrten Fläche 14 der Halteplatte 4 werden die Enden der Faszienhaltefäden durchgeführt und dann durch Verdrehen des Exzenters mittels der Handhabe 12 kontinuierlich stärker werdend festgeklemmt. In der Offenlage wird die Stellung des Exzenters 13 zum Beispiel durch Rasten und eine eingreifende abgefederte Kugel fixiert.

Durch die vorbeschriebene Anordnung ist es möglich, die Fixiervorrichtung mittels der Faszienhaltefäden leicht und bequem in der Bauchdeckenöffnung festzulegen. Der Arzt ist nunmehr in der Lage, die Trokarhülse in axiale Richtung durch die Fixiervorrichtung zu verschieben, um etwaige andere erforderliche Stellungen der Trokarhülse zu erreichen. Die Fixiervorrichtung selbst bleibt durch die Faszienhaltefäden unverändert in ihrer festgelegten Lage.

Die Fixiervorrichtung 2,4 ist im übrigen gegen die Trokarhülse 1 durch ein Dichtelement 15 abgedichtet, das durch eine aufschraubbare Kappe 16 über einen Druckring 7 in seiner Lage festgehalten wird.

Um die Fixiervorrichtung 2,4 für Trokarhülsen verschiedener Durchmesser verwenden zu können, wird das Dichtelement 15 mit Druckring 17 nach Lösen der Kappe 16 entfernt und es wird in den zentralen Kanal 3 eine den Durchgang der Fixiervorrichtung einengende Reduzierhülse 18,19 eingesetzt, die wieder durch die Schraubkappe 16 festgelegt wird (Figur 3). Diese Hülse 18,19 weist an ihrem oberen Ende eine den Druckring 17 ersetzende Erweiterung auf. Der Spannring 8 wird in der Reduzierhülse 18 mittels der Zylinderhülse 19 lose festgelegt. Der zentrale Kanal 3 der Fixiervorrichtung 2,4 bzw. die Reduzierhülse 18,19 wird oben durch ein Dichtelement 15 bzw. eine Gummikappe 20 mit mittlerem Durchgang verschlossen, die sich mit dem Lochrand dichtend gegen die Trokarhülse 1 legt und das Austreten von Gasen aus der Bauchhöhle verhindert.

## Patentansprüche

1. Fixiervorrichtung (2, 4) mit Klemmvorrichtungen (9) zum Festlegen von Faszienhaltefäden und mit einem Dichtkegel (2) für die offene Laparoskopie, der einen axialen Durchgang zum Durchführen und Festlegen einer ein Laparoskop aufnehmenden Trokarhülse (1) aufweist, dadurch gekennzeichnet, daß der Dichtkegel (2) eine an dessen proximalseitigem Ende lösbar festlegbare Halteplatte (4) mit mehreren manuell zu betätigenden Klemmvorrichtungen (9) zum lösbaren Festlegen der Enden von Faszienhaltefäden aufweist, und daß der Dichtkegel (2) und die Halteplatte (4) mit den Klemmvorrichtungen (9) gemeinsam mittels einer durch einen Handhebel (6) radial verschiebbaren, auf einen Spannring (8) wirkenden Druckstange (5) mit der Trokarhülse (1) lösbar festlegbar sind.

2. Fixiervorrichtung (2, 4) nach Anspruch 1, dadurch gekennzeichnet, daß zum Festklemmen des Dichtkegels (2) auf der Trokarhülse (1) der quer zur Druckstange (5) in einer Kulisse verschwenkbar gelagerte Handhebel (6) über eine Gewindebohrung mit einem Gewinde der Druckstange (5) derart im Eingriff steht, daß beim Verschwenken des Handhebels (6) der Spannring (8) über die Druckstange (5) und ein Ausgleichselement (5a) gegen die Trokarhülse (1) verschwenkt wird.

3. Fixiervorrichtung (2, 4) nach Anspruch 1, dadurch gekennzeichnet, daß die Klemmvorrichtung (9) für die Enden der Faszienhaltefäden je aus einem mit einer drehbar gelagerten Welle (11) verbundenen, den Abstand zur Flache (14) der sich an den Kegel anschließenden Halteplatte (4) kontinuierlich verringernden Exzenter (13) besteht, der über eine an der Welle angreifenden Handhabe (12) betätigbar ist.

4. Fixiervorrichtung (2, 4) nach Anspruch 3, dadurch gekennzeichnet, daß der Exzenter (13) auf seiner Oberfläche mit Querriefen oder dergleichen versehen ist.

5. Fixiervorrichtung (2, 4) nach einem der Ansprüche 1 bis 4, dadurch gekennzeichnet, daß der axiale Durchgang (3) des Dichtkegels (2) in einer oberen zylindrischen Erweiterung ein elastisches Dichtelement (15) aufweist, das durch eine starre Ringkappe (16) über einen Druckring (17) axial festlegbar ist.

6. Fixiervorrichtung (2, 4) nach einem der Ansprüche 1 bis 5, dadurch gekennzeichnet, daß anstelle des Dichtelementes (15), des Druckringes (17) in der oberen zylindrischen Ringerweiterung und des darunter angeordneten Spannringes (8) die Ringschulter einer in den axialen Durchgang (3) einsetzbaren Reduzierhülse (18) axial festlegbar ist, und die Reduzierhülse (18) eine einen unterbrochenen Spannring (8) axial lose abstützende zylinderförmige Ausnehmung aufweist und das obere offene Ende der Reduzierhülse durch eine Gummikappe (20) mit einer mittleren Durchbrechung geschlossen ist.

7. Fixiervorrichtung (2, 4) nach einem der Ansprüche 1 bis 6, dadurch gekennzeichnet, daß der Dichtkegel (2) und die Halteplatte (4) gasdicht lösbar miteinander festlegbar sind.

## Claims

1. Fixing device (2, 4) with clamping devices (9) for fixing fascia retaining threads and with a sealing cone (2) for open laparoscopy, which has an axial passage for guiding and fixing a trocar sleeve (1) receiving a laparoscope, characterised in that the sealing cone (2) has a retaining plate (4) which can be releasably fixed to its proximal-side end and having several manually actuated clamping devices (9) for releasably fixing the ends of fascia retaining threads, and in that the sealing cone (2) and the retaining plate (4) with the clamping devices (9) can all be releasably fixed to the trocar sleeve (1) by means of a pressure rod (5) acting on a tension ring (8) and which can be radially displaced by means of a lever (6).

2. Fixing device (2, 4) according to claim 1, characterised in that the lever (6) mounted pivotably in a connecting member transversely to the pressure rod (5) engages with a thread of the pressure rod (5) by means of a threaded bore to clamp the sealing cone (2) on the trocar sleeve (1), such that when pivoting the lever (6), the tension ring (8) is pivoted over the pressure rod (5) and a compensation element (5a) is pivoted against the trocar sleeve (1).

3. Fixing device (2, 4) according to claim 1, characterised in that the clamping device (9) for the ends of the fascia retaining threads comprises in each case an eccentric (13), which continuously reduces the distance to the surface (14) of the retaining plate (4) connected to the cone and is connected to a rotatably mounted shaft (11), and which can be actuated by means of a handle (12) acting on the shaft.

4. Fixing device (2, 4) according to claim 3, characterised in that the eccentric (13) is provided with transverse grooves or the like on its surface.

5. Fixing device (2, 4) according to one of claims 1 to 4, characterised in that the axial passage (3) of the sealing cone (2) has a resilient sealing element (15) in an upper cylindrical enlargement which can be fixed axially by means of a rigid ring cap (16) over a pressure ring (17).

6. Fixing device (2, 4) according to one of claims 1 to 5, characterised in that instead of the sealing element (15), the pressure ring (17) in the upper cylindrical ring enlargement and the tension ring (8) arranged therebelow, the ring shoulder of a reducing sleeve (18) which can be inserted in the axial passage (3) can be fixed axially, and the reducing sleeve (18) has a cylindrical recess loosely supporting a discontinued tension ring (8) axially and the upper open end of the reducing sleeve is sealed by means of a rubber cap (20) with a central perforation.

7. Fixing device (2, 4) according to one of claims 1 to 6, characterised in that the sealing cone (2) and the retaining plate (4) can be fixed to one another releasably in a gas-tight manner.

## Revendications

1. Dispositif de fixation (2,4) avec des dispositifs de serrage (9) pour fixer des fils de maintien de faisceaux et avec un joint conique (2) pour laparoscopie, qui présente un passage axial pour enfiler et fixer une douille de trocart (1) recevant un laparoscope, caractérisé en ce que le joint conique (2) présente une plaque de maintien (4) fixable de manière amovible à son extrémité proximale et qui est équipée de plusieurs dispositifs de serrage manuels (9) pour fixer de manière amovible les extrémités des fils de maintien de faisceaux et le joint conique (2) et la plaque de maintien (4) peuvent être fixés de manière amovible par les dispositifs de serrage (9), conjointement via une tige de compression (5) agissant sur une bague d'encastrement (8) et déplaçable radialement à l'aide d'un levier (6), avec la douille (1) du trocart.

2. Dispositif de fixation (2,4) selon la revendication 1, caractérisé en ce que, pour fixer le joint conique (2) sur la douille (1) du trocart, le levier (8) monté de manière à pouvoir pivoter transversalement par rapport à la tige de compression (5) dans une coulisse coopère via un alésage fileté avec un filetage de la tige de compression (5) en sorte que, lors du pivotement du levier (6), la bague d'encastrement (8) soit soumise à un pivotement contre la douille (1) du trocart via la tige de piston (5) et un élément de compensation (5a).

3. Dispositif de fixation (2,4) selon la revendication 1, caractérisé en ce que les dispositifs de serrage (9) pour les extrémités des fils de maintien de faisceaux sont constitués respectivement d'une excentrique (13), reliée à un arbre (11) à rotation, réduisant en continu l'écart vis-à-vis de la surface (14) de la plaque de soutien (4) raccordée au cône, cette excentrique pouvant être commandée via une manette (12) s'engrenant sur l'arbre.

4. Dispositif de fixation (2,4) selon la revendication 3, caractérisé en ce que l'excentrique (13) est dotée à sa surface de cannelures transversales ou d'éléments analogues.

5. Dispositif de fixation (2,4) selon l'une quelconque des revendications 1 à 4, caractérisé en ce que le passage axial (3) du joint conique (2) présente dans son évasement cylindrique supérieur un élément d'étanchéité élastique (15) qui peut être fixé axialement par une coiffe annulaire rigide (16) via une bague de compression (17).

6. Dispositif de fixation (2,4) selon l'une quelconque des revendications 1 à 5, caractérisé en ce que, au lieu de l'élément d'étanchéité (15), de la bague de compression (17) dans l'extension annulaire cylindrique supérieure et de la bague d'encastrement (8) agencée en dessous, l'épaulement annulaire d'une douille de réduction (18) est susceptible d'être monté dans le passage axial (3) et peut être fixé axialement, et la douille de réduction (18) présente un évidement cylindrique s'appuyant de manière lâche axialement sur la bague d'encastrement (8) interrompue et l'extrémité ouverte supérieure de la bague de réduction est fermée par une coiffe de caoutchouc (20) avec une découpe centrale.

7. Dispositif de fixation (2,4) selon l'une quelconque des revendications 1 à 6, caractérisé en ce que le joint conique (2) et la plaque de maintien (4) peuvent être fixés l'un à l'autre de manière amovible et de manière étanche aux gaz.
